# EUROPEAN PATENT APPLICATION

(11) **EP 4 187 246 A1**
(43) Date of publication of application: **31.05.2023**
(21) Application number: 21847073.0
(22) Date of filing: 20.07.2021
(51) Int. Cl.: G01N 33/53, C12M 1/34

(54) **METHOD FOR ASSISTING DIAGNOSIS OF INFLAMMATORY BOWEL DISEASE**

(30) Priority: 22.07.2020 JP 2020124881
(71) Applicant: FUJIFILM Corporation, Tokyo 106-8620 (JP); Osaka University, Suita-shi Osaka 565-0871 (JP)
(72) Inventor: MIYOSHI, Eiji, Suita-shi, Osaka 565-0871 (JP); MORISHITA, Koichi, Suita-shi, Osaka 565-0871 (JP); SHINZAKI, Shinichiro, Suita-shi, Osaka 565-0871 (JP); MOTOOKA, Kei, Suita-shi, Osaka 565-0871 (JP); KIDOWAKI, Kayoko, Amagasaki-shi, Hyogo 661-0963 (JP); TAMURA, Ikumi, Amagasaki-shi, Hyogo 661-0963 (JP); DATE, Mutsuhiro, Amagasaki-shi, Hyogo 661-0963 (JP)
(74) Representative: Owen, Deborah Jane
(86) International application number: PCT/JP2021/027099
(87) International publication number: WO 2022/019298

(57) **Abstract**

An object of the present invention is to provide a method of assisting diagnosis of inflammatory bowel disease, which can specifically determine inflammatory bowel disease.

The present invention relates to "a method of assisting diagnosis of inflammatory bowel disease, the method including subjecting a subject-derived specimen to a reduction treatment and subsequently measuring a human prohaptoglobin amount in the specimen by using an antibody 1 which is an antibody that specifically binds to an amino acid sequence set forth in SEQ ID NO: 1, and determining that a subject has inflammatory bowel disease by using the human prohaptoglobin amount as an indicator, and relates to an examination kit for assisting diagnosis of inflammatory bowel disease, including the antibody 1 a reducing agent".

## Description

### BACKGROUND OF THE INVENTION

### 1. Description of the Related Art

Inflammatory bowel disease (IBD) is a refractory chronic inflammatory disease in which the intestinal tract is repeatedly inflamed, which is roughly classified into ulcerative colitis and Crohn's disease, and the number of patients thereof has been increasing in recent years. The diagnosis of inflammatory bowel disease is carried out by colonoscopy, gastrointestinal contrast radiography, a histopathological examination, or the like. Since patients suffering from inflammatory bowel disease have a high risk of developing cancer as compared with those not suffering from inflammatory bowel disease, it is necessary to continue the examination for a long period of time. However, colonoscopy is highly invasive, and a physical burden on a patient who is a subject is large in a case of constantly monitoring a pathological condition by endoscopy.

Therefore, a diagnosis using a biomarker that makes it possible to carry out a non-invasive diagnosis of inflammatory bowel disease has also been carried out.

For example, human haptoglobin is a reactive protein in the acute stage, which is synthesized in the liver, and it is known that the blood concentration of human haptoglobin varies in ulcerative colitis and Crohn's disease (JP2009-526233A and JP2012-529508A). The human haptoglobin concentrations in sera of patients with ulcerative colitis and patients with Crohn's disease have also been measured (JP2012-507723A).

### SUMMARY OF THE INVENTION

However, known biomarkers for determining inflammatory bowel disease do not reflect intestinal inflammation specific for inflammatory bowel disease.

The present invention has been made in consideration of the above-described situation, and an object of the present invention is to provide a method of assisting diagnosis of inflammatory bowel disease, which can specifically determine inflammatory bowel disease.

Recently, it has been reported that there is a possibility that prohaptoglobin has a biological function different from that of mature haptoglobin. As a result of diligent studies to find an excellent biomarker that enables the determination of inflammatory bowel disease with high accuracy, the inventors of the present invention found that in a case of carrying out the measurement of human prohaptoglobin by using an antibody that specifically binds to a region of an amino acid sequence set forth in SEQ ID NO: 1, the region being contained in the α chain of human prohaptoglobin, the inflammatory bowel disease can be determined with high specificity, thereby completing the present invention.

The present invention has the following configurations.
[1] A method of assisting diagnosis of inflammatory bowel disease, the method comprising:
   subjecting a subject-derived specimen to a reduction treatment and subsequently measuring a human prohaptoglobin amount in the specimen by using an antibody 1 which is an antibody that specifically binds to an amino acid sequence set forth in SEQ ID NO: 1; and
   determining that a subject has inflammatory bowel disease by using the human prohaptoglobin amount as an indicator.
[2] The method of assisting diagnosis of inflammatory bowel disease according to [1], in which the determination is to determine that a subject has inflammatory bowel disease in a case where the human prohaptoglobin amount is equal to or larger than a reference value.
[3] The method of assisting diagnosis of inflammatory bowel disease according to [1] or [2], in which the measurement includes the following steps (A-1) to (A-4);
   (A-1) the step of subjecting a subject-derived specimen to a reduction treatment,
   (A-2) the step of separating human prohaptoglobin from the specimen obtained in the step (A-1),
   (A-3) the step of bringing the human prohaptoglobin obtained in the step (A-2) into contact with the antibody 1 to form a complex of the human prohaptoglobin and the antibody 1, and
   (A-4) the step of measuring an amount of the complex obtained in the step (A-3) to measure a human prohaptoglobin amount.
[4] The method of assisting diagnosis of inflammatory bowel disease according to [3], in which the step (A-2) is carried out by an electrophoresis method.
[5] The method of assisting diagnosis of inflammatory bowel disease according to [1] or [2], in which the measurement includes the following steps (B-1) to (B-4);
   (B-1) the step of subjecting a subject-derived specimen to a reduction treatment,
   (B-2) the step of bringing the specimen obtained in the step (B-1) into contact with the antibody 1 to form a complex of human prohaptoglobin in the specimen and the antibody 1,
   (B-3) the step of separating the complex obtained in the step (B-2), and
   (B-4) the step of measuring an amount of the complex separated in the step (B-3) to measure a human prohaptoglobin amount.
[6] The method of assisting diagnosis of inflammatory bowel disease according to [5], in which the step (B-3) is carried out by an electrophoresis method.
[7] The method of assisting diagnosis of inflammatory bowel disease according to [1] or [2], in which the measurement includes the following steps (C-1) to (C-3);
   (C-1) the step of subjecting a subject-derived specimen to a reduction treatment,
   (C-2) the step of bringing the specimen obtained in the step (C-1) into contact with the antibody 1 and an antibody 2, which is an antibody that recognizes a β chain of human prohaptoglobin, to form a complex of human prohaptoglobin in the specimen, the antibody 1, and the antibody 2, and
   (C-3) the step of measuring an amount of the complex obtained in the step (C-2) to measure a human prohaptoglobin amount.
[8] The method of assisting diagnosis of inflammatory bowel disease according to [7], in which any one of the antibody 1 or the antibody 2 is immobilized on an insoluble carrier, and the other thereof is labeled with a labeling substance.
[9] The method of assisting diagnosis of inflammatory bowel disease according to [1] or [2], in which the measurement includes the following steps (D-1) to (D-4);
   (D-1) the step of subjecting a subject-derived specimen to a reduction treatment,
   (D-2) the step of bringing the specimen obtained in the step (D-1), the antibody 1, an antibody 1-2 which is an antibody that recognizes an α chain of human prohaptoglobin, the antibody 1-2 having an epitope different from an epitope recognized by the antibody 1, and an antibody 2 which is an antibody that recognizes a β chain of human prohaptoglobin, into contact with each other, to form a complex of prohaptoglobin in the specimen, the antibody 1, the antibody 1-2, and the antibody 2,
   (D-3) the step of separating the complex of the prohaptoglobin, the antibody 1, the antibody 1-2, and the antibody 2, where the complex is obtained in the step (D-2) , and
   (D-4) the step of measuring an amount of the complex separated in the step (D-3) to measure a human prohaptoglobin amount.
[10] The method of assisting diagnosis of inflammatory bowel disease according to [9], in which the step (D-3) is carried out by a capillary electrophoresis method.
[11] The method of assisting diagnosis of inflammatory bowel disease according to any one of [1] to [10], in which the subject-derived specimen is serum, blood plasma, or whole blood.
[12] A method of obtaining data for assisting diagnosis of inflammatory bowel disease, the method comprising:
   subjecting a subject-derived specimen to a reduction treatment and subsequently measuring a human prohaptoglobin amount in the specimen by using an antibody 1 which is an antibody that specifically binds to an amino acid sequence set forth in SEQ ID NO: 1.
[13] An examination kit for assisting diagnosis of inflammatory bowel disease, comprising:
   an antibody 1 which is an antibody that specifically binds to an amino acid sequence set forth in SEQ ID NO: 1; and
   a reducing agent.
[14] The examination kit for assisting diagnosis according to [13], further comprising an antibody 2 which is an antibody that recognizes a β chain of human prohaptoglobin.
[15] The examination kit for assisting diagnosis according to [14], further comprising an antibody 1-2 which is an antibody that recognizes an α chain of human prohaptoglobin, where the antibody 1-2 has an epitope different from an epitope recognized by the antibody 1.
[16] A device for assisting diagnosis of inflammatory bowel disease, comprising:
   a measurement unit that measures an amount of human prohaptoglobin in a subject-derived specimen.
[17] The device for assisting diagnosis of inflammatory bowel disease according to [16], further comprising a determination unit that determines whether or not a measured value obtained by the measurement in the measurement unit is equal to or larger than a reference value.
[18] The method of obtaining data for assisting diagnosis of inflammatory bowel disease according to [12], further comprising determining whether or not the human prohaptoglobin amount is equal to or larger than a reference value.
[19] The method of obtaining data for assisting diagnosis of inflammatory bowel disease according to [12] or [18], in which the measurement includes the following steps (A-1) to (A-4);
   (A-1) the step of subjecting a subject-derived specimen to a reduction treatment,
   (A-2) the step of separating human prohaptoglobin from the specimen obtained in the step (A-1),
   (A-3) the step of bringing the human prohaptoglobin obtained in the step (A-2) into contact with the antibody 1 to form a complex of the human prohaptoglobin and the antibody 1, and
   (A-4) the step of measuring an amount of the complex obtained in the step (A-3) to measure a human prohaptoglobin amount.
[20] The method of obtaining data for assisting diagnosis of inflammatory bowel disease according to [19], in which the step (A-2) is carried out by an electrophoresis method.
[21] The method of obtaining data for assisting diagnosis of inflammatory bowel disease according to [12] or [18], in which the measurement includes the following steps (B-1) to (B-4);
   (B-1) the step of subjecting a subject-derived specimen to a reduction treatment,
   (B-2) the step of bringing the specimen obtained in the step (B-1) into contact with the antibody 1 to form a complex of human prohaptoglobin in the specimen and the antibody 1,
   (B-3) the step of separating the complex obtained in the step (B-2), and
   (B-4) the step of measuring an amount of the complex separated in the step (B-3) to measure a human prohaptoglobin amount.
[22] The method of obtaining data for assisting diagnosis of inflammatory bowel disease according to [21],
   in which the step (B-3) is carried out by an electrophoresis method.
[23] The method of obtaining data for assisting diagnosis of inflammatory bowel disease according to [12] or [18],
   in which the measurement includes the following steps (C-1) to (C-3);
   (C-1) the step of subjecting a subject-derived specimen to a reduction treatment,
   (C-2) the step of bringing the specimen obtained in the step (C-1) into contact with the antibody 1 and an antibody 2, which is an antibody that recognizes a β chain of human prohaptoglobin, to form a complex of human prohaptoglobin in the specimen, the antibody 1, and the antibody 2, and
   (C-3) the step of measuring an amount of the complex obtained in the step (C-2) to measure a human prohaptoglobin amount.
[24] The method of obtaining data for assisting diagnosis of inflammatory bowel disease according to [23], in which any one of the antibody 1 or the antibody 2 is immobilized on an insoluble carrier, and the other thereof is labeled with a labeling substance.
[25] The method of obtaining data for assisting diagnosis of inflammatory bowel disease according to [12] or [18], in which the measurement includes the following steps (D-1) to (D-4);
   (D-1) the step of subjecting a subject-derived specimen to a reduction treatment,
   (D-2) the step of bringing the specimen obtained in the step (D-1), the antibody 1, an antibody 1-2 which is an antibody that recognizes an α chain of human prohaptoglobin, the antibody 1-2 having an epitope different from an epitope recognized by the antibody 1, and an antibody 2 which is an antibody that recognizes a β chain of human prohaptoglobin, into contact with each other, to form a complex of prohaptoglobin in the specimen, the antibody 1, the antibody 1-2, and the antibody 2,
   (D-3) the step of separating the complex of the prohaptoglobin, the antibody 1, the antibody 1-2, and the antibody 2, where the complex is obtained in the step (D-2), and
   (D-4) the step of measuring an amount of the complex separated in the step (D-3) to measure a human prohaptoglobin amount.
[26] The method of obtaining data for assisting diagnosis of inflammatory bowel disease according to [25],
   in which the step (D-3) is carried out by a capillary electrophoresis method.
[27] The method of obtaining data for assisting diagnosis of inflammatory bowel disease according to any one of [12] or [18] to [26], in which the subject-derived specimen is serum, blood plasma, or whole blood.

The method of assisting diagnosis of inflammatory bowel disease according to the aspect of the present invention can be used as a method of assisting diagnosis by a doctor or the like.

In addition, all the above methods are carried out in vitro.

According to the present invention, it is possible to obtain data for assisting diagnosis of inflammatory bowel disease according to the aspect of the present invention using the antibody 1 according to the aspect of the present invention. In addition, the diagnosis of inflammatory bowel disease, which is highly specific in that inflammatory bowel disease can be distinguished from gastrointestinal cancer and a state of a healthy subject, can be assisted by using the data.

### BRIEF DESCRIPTION OF THE DRAWINGS

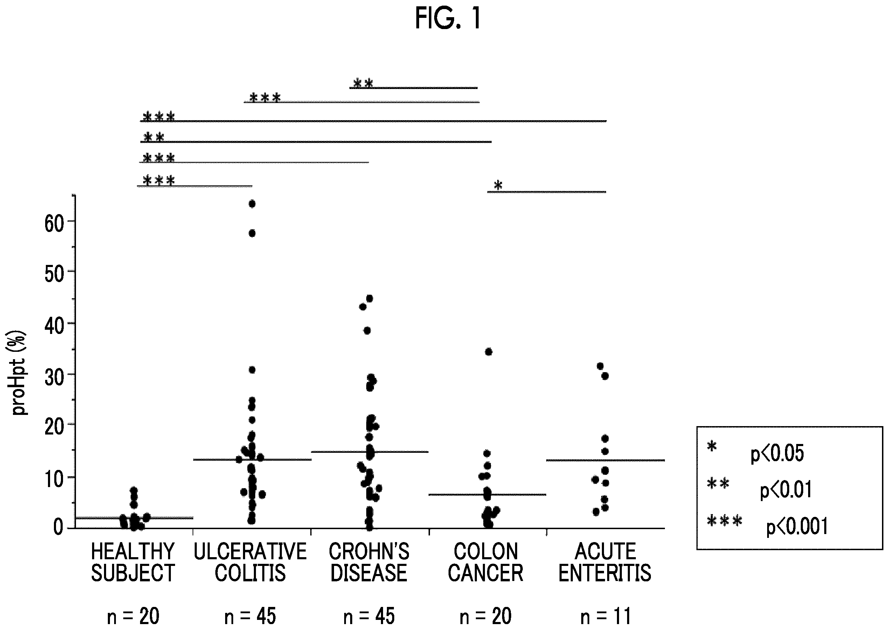

Fig. 1 shows a graph of the distribution of human prohaptoglobin amounts (proHpt (%)), obtained by measurements using sera of healthy subjects, patients with ulcerative colitis, patients with Crohn's disease, patients with colon cancer, and patients with acute enteritis, which are obtained in Example 1.

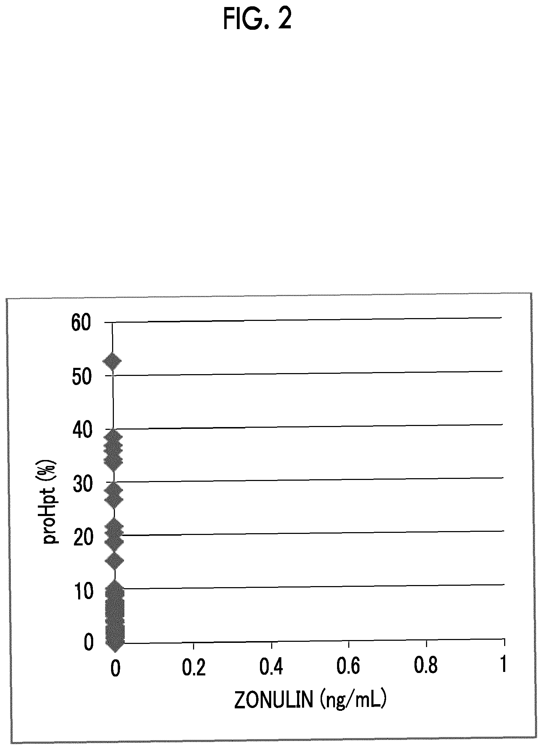

Fig. 2 is a correlation graph showing a correlation between results of measurements of human prohaptoglobin using sera of patients with ulcerative colitis, obtained in Example 2 by a method according to the present invention, and results of measurements of human prohaptoglobin using a known zonulin measurement kit.

### DESCRIPTION OF THE PREFERRED EMBODIMENTS

The present invention relates to a method of assisting diagnosis of inflammatory bowel disease using human prohaptoglobin as an indicator, a method of obtaining data for assisting the diagnosis, and a kit that is used for the methods.

### <1. Inflammatory bowel disease>

The inflammatory bowel disease according to the present invention includes ulcerative colitis and Crohn's disease, which are generally classified as inflammatory bowel disease. It is preferably ulcerative colitis and Crohn's disease.

### <2. Human prohaptoglobin>

Prohaptoglobin is a heterodimer in which two subunits of an α chain and a β chain are bound to each other, and it is a precursor of haptoglobin. Prohaptoglobin is cleaved into an α chain and a β chain by the serine protease activity of C1RLP, and both chains become haptoglobin which is linked through a disulfide bond. The prohaptoglobin according to the embodiment of the present invention refers to a human prohaptoglobin derived from a human.

The human prohaptoglobin according to the embodiment of the present invention is hereinafter abbreviated as "proHpt".

Haptoglobin is a liver-derived glycoprotein present in the blood of mammals, and it is generally known that the blood concentration of haptoglobin decreases during hemolysis since haptoglobin binds to hemoglobin liberated during hemolysis. The haptoglobin according to the embodiment of the present invention refers to a human haptoglobin derived from a human. The human haptoglobin according to the embodiment of the present invention is hereinafter abbreviated as "Hpt".

There are two types of α chains of Hpt, an α1 chain and an α2 chain. The α1 chain is about 10 kDa and the α2 chain is about 18 kDa. The β chain is about 39 kDa. Hpt is classified into three types: an Hpt1-1 type ((α1β)₂), an Hpt2-1 type ((α1β)ₘ(α2β)ₙ), and an Hpt2-2 type ((α2β)ₙ). Here, m and n are integers of 1 or more and may be the same or different from each other. The β chains of the three types are the same.

### <3. Method of assisting diagnosis of inflammatory bowel disease>

The method of assisting diagnosis of inflammatory bowel disease according to the present invention is a method of assisting diagnosis of inflammatory bowel disease, including "subjecting a subject-derived specimen to a reduction treatment and subsequently measuring a proHpt amount in the specimen by using an antibody 1 which is an antibody that specifically binds to an amino acid sequence set forth in SEQ ID NO: 1, and determining that a subject has inflammatory bowel disease using the proHpt amount as an indicator".

In addition, the present invention includes a method of obtaining data for assisting diagnosis of inflammatory bowel disease, the method characterized by being subjecting a subject-derived specimen to a reduction treatment in this way and subsequently measuring a proHpt amount in the specimen by using an antibody 1 according to an embodiment of the present invention.

### 1. Specimen

In the present invention, examples of the specimen that is used for measuring the proHpt amount in the specimen include body fluids derived from a human as a subject, such as serum, blood plasma, blood (whole blood), a pancreatic fluid, saliva, a lymph fluid, and a spinal fluid, intestinal tissues such as the large intestine or the small intestine, an extract of the tissue, a tissue section of the tissue, a washing solution of the tissue, and specimens prepared from these. Among them, serum, blood plasma, or blood (whole blood) is preferable. Serum or blood plasma is more preferable, and serum is particularly preferable.

### 2. Measurement method for proHpt

In the method of assisting diagnosis of inflammatory bowel disease according to the present invention, the proHpt amount is measured in order to obtain data for assisting diagnosis of inflammatory bowel disease. However, the measurement method for the proHpt amount according to the step is "a method of measuring a proHpt amount in a subject-derived specimen by subjecting a subject-derived specimen to a reduction treatment and subsequently using an antibody 1 which is an antibody that specifically binds to an amino acid sequence set forth in SEQ ID NO: 1".

### (I) Reduction treatment

As described above, in Hpt, the α chain and the β chain are linked through a disulfide bond. Accordingly, in a case where Hpt is subjected to a reduction treatment to cleave the disulfide bond that links the α chain and the β chain of the Hpt, the Hpt is dissociated into the α chain and the β chain. On the other hand, the α chain and β chain of the proHpt are not dissociated even after the reduction treatment.

In the measurement method for proHpt according to the embodiment of the present invention, a subject-derived specimen is subjected to a reduction treatment with, for example, a reducing agent to dissociate in Hpt in the specimen into the α chain and the β chain. Next, the proHpt amount in the specimen subjected to the reduction treatment may be measured.

The reducing agent that is used in the above method is not particularly limited as long as it is a drug that is capable of reducing and cleaving the disulfide bond that links the α chain and the β chain of Hpt. Examples thereof include an SH group compound or a salt thereof, a thiourea derivative, and a phosphine derivative.

More specific examples thereof include dithiothreitol (DTT), glutathione, a tris(2-carboxyethyl)phosphine hydrochloride (TCEP-HCl), dithioerythritol (DTE), α-thioglycerol, a 2-mercaptoethylamine hydrochloride, a 2-aminoethylisothiouronium bromide hydrobromide, thioredoxin, glutathione reductase, 2-mercaptoethanol, 3-mercapto-1,2-propanediol, tributyl phosphine, NaBH₄, NADPH, thioglycolic acid, sodium sulfite, sodium hydrogen sulfite, sodium metabisulfite, and nitrogen monoxide.

Dithiothreitol (DTT), 2-mercaptoethanol, or 3-mercapto-1,2-propanediol is preferable.

The reducing agent is preferably used as a form of a solution thereof obtained by being dissolved in a proper buffer solution. Examples of the buffer solution that is used for such an intended purpose include a Tris buffer solution, a phosphate buffer solution, a borate buffer solution, and a Good buffer solution.

The concentration of the reducing agent is 0.001 to 100 w/v% and preferably 0.01 to 50 w/w% in terms of a concentration in a case of being mixed with a subject-derived specimen. Alternatively, it is 20 to 500 mM and preferably 30 to 400 mM.

The time of the reduction reaction in which a subject-derived specimen is treated with a reducing agent is 1 minute to 72 hours, preferably 1 minute to 12 hours, and more preferably 1 minute to 100 minutes.

The temperature at the time of the reduction reaction is 0°C to 120°C and preferably 25°C to 100°C.

As a method of subjecting a subject-derived specimen to a reduction treatment, a subject-derived specimen may be mixed with a reducing agent or a solution of the reducing agent so that the subject-derived specimen is under the above-described conditions.

### (II) Antibody 1 according to embodiment of present invention

The "antibody that specifically binds to an amino acid sequence set forth in SEQ ID NO: 1", which is used in the measurement method for proHpt according to the embodiment of the present invention is hereinafter abbreviated as the "antibody 1 according to the embodiment of the present invention" or simply the "antibody 1".

The antibody 1 according to the embodiment of the present invention includes "an antibody that specifically recognizes a primary structure of the amino acid sequence set forth in SEQ ID NO: 1 of the α chain of proHpt" or "an antibody that specifically recognizes a three-dimensional structure within a region of the amino acid sequence forth in SEQ ID NO: 1 of the α chain of proHpt".

That is, the antibody 1 according to the embodiment of the present invention binds to a region of 6 amino acids set forth in SEQ ID NO: 1 of the α chain of proHpt.

The antibody 1 according to the embodiment of the present invention may be an antibody having the above-described characteristics, and it may be a monoclonal antibody or a polyclonal antibody. A monoclonal antibody is more preferable. In addition, it may be a commercially available product or an antibody appropriately prepared according to a conventional method. Further, it is optional that these are used alone or in a combination thereof in the measurement of proHpt using the antibody 1 according to the embodiment of the present invention, which will be described later.

It is noted that the amino acid sequence of the α1 chain of Hpt is set forth in SEQ ID NO: 2. Here, the amino acid sequence set forth in SEQ ID NO: 1 (QCKNYY) is contained at a position between the 51st to 56th sites from the N-terminal thereof.

The amino acid sequence of the α2 chain of Hpt is set forth in SEQ ID NO: 3. Here, the amino acid sequence set forth in SEQ ID NO: 1 is contained at two positions of a position between the 51st to 56th sites and a position between the 110th to 115th sites from the N-terminal thereof.

That is, the α chain of Hpt also has the amino acid sequence of SEQ ID NO: 1. As a result, an antibody that binds to the region of SEQ ID NO: 1 of the α chain of Hpt can also be used as the antibody 1 according to the embodiment of the present invention.

The antibody 1 according to the embodiment of the present invention may be an antigen-binding fragment of the antibody 1. The antigen-binding fragment means an antibody fragment having an antigen-binding site. Specific examples thereof include fragments of the antibody 1, such as Fab, Fab', F(ab')₂, Fv, Fd, single chain Fv (scFv), disulfide bonded Fv (sdFv), VL, VH, a diabody ((VL-VH)₂ or (VH-VL)₂), a triabody (a trivalent antibody), a tetrabody (a tetravalent antibody), a minibody ((scFV-CH₃)₂), IgG-delta-CH₂, scFv-Fc, and (scFv)₂-Fc, which specifically bind to the amino acid sequence set forth in SEQ ID NO: 1.

Examples of the polypeptide that is used as an immunogen (an antigen) in order to obtain the antibody 1 according to the embodiment of the present invention, include (a) a polypeptide having an amino acid sequence of the α chain of Hpt (for example, the amino acid sequence set forth in SEQ ID NO: 2 or SEQ ID NO: 3), (b) a polypeptide having a partial sequence of the amino acid sequence of the α chain of Hpt (for example, the amino acid sequence set forth in SEQ ID NO: 2 or SEQ ID NO: 3), which contains the amino acid sequence set forth in SEQ ID NO: 1, or (c) proHpt (full length).

The above-described (a), (b), or (c), which is used as an immunogen in order to obtain the antibody 1 according to the embodiment of the present invention, can be produced by a general chemical manufacturing method according to the amino acid sequence thereof. The polypeptide can be obtained, for example, according to a conventional chemical synthesis method such as a fluorenylmethyloxycarbonyl method (an Fmoc method) or a t-butyloxycarbonyl method (a tBoc method). In addition, chemical synthesis can also be carried out using a commercially available peptide synthesizer. In addition, a manufactured product obtained by being outsourced to a manufacturer that manufactures a synthetic peptide product may be used.

The above-described (a) Hpt (full length) that is used as an immunogen in order to obtain the antibody 1 according to the embodiment of the present invention can be also obtained by extraction and purification with a method using, for example, an anti-Hpt antibody column, from a culture solution or culture supernatant of a cancer cell line, such as a culture supernatant of cells obtained by introducing the Hpt gene into a human colon cancer cell line HCT116 (ATCC) to stably overexpress it. Alternatively, a purified product or the like of Hpt, which is commercially available, can be used.

In addition, the above-described (c) proHpt (fulllength) that is used as an immunogen can be also obtained, according to a previous report (Mi-Kyung Oh, et al., FEBS letters vol. 589, pp. 1009-1017, 2015), by constructing an expression vector of Hpt in which an amino acid mutation has been introduced in a portion to be cleaved by C1RL, overexpressing the Hpt in a proper host cell such as HEK293 cells, subsequently recovering the culture supernatant, and then carrying out recovery and purification by affinity column chromatography using an Hpt-binding carrier.

The purification of the antigenic protein serving as an immunogen may be carried out by a method known per se, for example, a combination of several chromatography techniques such as affinity chromatography using Sepharose beads coated with an anti-Hpt antibody or an anti-Hpt α chain antibody.

In the method of obtaining a polyclonal antibody 1 according to the embodiment of the present invention, it suffices that, the antibody is produced according to a conventional method in which animals, for example, horses, cows, sheep, rabbits, goats, guinea pigs, rats, and mice are immunized with an immunogen obtained by such a method as described above, according to a conventional method [for example, a method described in Introduction to Immunological Experiments, 2nd Printing, Nao Matsuhashi et al., Japan Scientific Societies Press, 1981, or the like], and an antibody that specifically binds to the amino acid sequence set forth in SEQ ID NO: 1 is selected according to a conventional method.

In addition, examples of the method of obtaining a monoclonal antibody 1 according to the embodiment of the present invention include the following method. That is, hybridomas are prepared by fusing, for example, immunosensitized cells such as splenocytes and lymphocytes of an animal, for example, a rat or a mouse, which has been immunized with the immunogen obtained by such a method as described above, and for example, cells having a property of permanently being proliferated, such as myeloma cells, according to the cell fusion technique (Nature, 256, 495, 1975) known per se developed by Keller and Milstein et al., and a hybridoma that produces a monoclonal antibody that specifically binds to the amino acid sequence represented by SEQ ID NO: 1 is selected. The selected hybridoma may be cultured in a culture medium or administered intraperitoneally to an animal to produce an antibody in ascites, and a monoclonal antibody of interest may be collected from the culture or the ascites. Alternatively, cells that produce an antibody having such a property as described above are prepared according to a method known per se (Eur. J. Immunol., 6, 511, 1976) to which a gene recombination technique or the like has been applied, and these cells may be cultured to collect the monoclonal antibody 1 of interest according to the embodiment of the present invention.

An example of the method of producing the antibody 1 according to the embodiment of the present invention will be described as below, taking a method of producing a monoclonal antibody as an example.

That is, an immunogen [(a) a polypeptide having an amino acid sequence of the α chain of the Hpt according to the embodiment of the present invention (for example, the amino acid sequence set forth in SEQ ID NO: 2 or SEQ ID NO: 3), (b) a polypeptide having a partial sequence of the amino acid sequence of the α chain of the Hpt according to the embodiment of the present invention (for example, the amino acid sequence set forth in SEQ ID NO: 2 or SEQ ID NO: 3), which contains the amino acid sequence set forth in SEQ ID NO: 1, or (c) proHpt (full length)] is mixed with an adjuvant such as a complete (or incomplete) Freund's adjuvant to prepare a suspension. To such suitable animals as described above, this suspension in terms of the amount of generally 1 ng to 10 mg of the antigen is administered, for immunization, subcutaneously, intravenously, or intraperitoneally, generally every 1 to 5 weeks and preferably every 2 to 5 weeks, and generally 2 to 10 times and preferably 3 to 8 times.

Three to four days after the final immunization, the spleen and the lymph nodes are aseptically excised from the immunized animal. The spleen is most preferable as the organ to be excised. From the excised spleen, splenocytes are prepared according to a conventional method. The obtained splenocytes and myeloma cells such as NS-1, Sp2, Sp2/0, or X63 are fused according to a conventional method. Examples of the cell fusion method include a method using polyethylene glycol and a cell electrofusion method. However, a method using polyethylene glycol is preferable because it is simple.

Next, according to a conventional method, a hybridoma is selected using a HAT medium.

The selected hybridoma is cultured, and the culture supernatant is collected. The culture supernatant is subjected to a general ELISA method, an indirect immunofluorescence method, a Western blot immunostaining method using a polyvinylidene difluoride (PVDF) membrane after SDS-polyacrylamide gel electrophoresis, or the like, to select a cell (a hybridoma) that specifically recognizes proHpt and produces an antibody that specifically binds to the amino acid sequence set forth in SEQ ID NO: 1.

Next, cloning by the limiting dilution method is carried out several times, and a cell in which a stable production of an antibody having a high titer has been recognized is selected as a hybridoma strain that produces the monoclonal antibody 1 according to the embodiment of the present invention.

By the above-described method, it is possible to obtain a hybridoma that produces the monoclonal antibody 1 of interest according to the embodiment of the present invention.

In order to obtain the monoclonal antibody 1 according to the embodiment of the present invention from the obtained hybridoma, the hybridoma obtained by the above method may be cultured, and the monoclonal antibody 1 may be purified from the obtained culture supernatant according to a conventional method.

Examples of the method of culturing a hybridoma include a method of forming ascites, in which a hybridoma is intraperitoneally administered to an animal, and a conventional method such as a cell culture method in which the hybridoma is cultured.

The purification of the monoclonal antibody from the culture supernatant or mouse ascites may be carried out by appropriately selecting and combining known methods such as an ammonium sulfate salting-out method, affinity chromatography, ion exchange chromatography, and molecular sieving chromatography.

Preferred specific examples of the antibody 1 according to the embodiment of the present invention include "10-7G2A" established in PCT/JP2020/020669 by the inventors of the present invention, which is a novel antibody 1 that specifically binds to the region of the amino acid sequence set forth in SEQ ID NO: 1 of the α chain of Hpt.

### (III) Measurement method for proHpt

In the method of assisting diagnosis of inflammatory bowel disease according to the present invention, in the step of measuring the proHpt amount in order to obtain the data that is used in the method, the proHpt amount in the specimen subjected to the reduction treatment may be measured using the antibody 1. Examples of the measurement method include enzyme immunoassay (EIA), radioimmunoassay (RIA), enzyme-linked immunosorbent assay (ELISA), fluoroimmunoassay (FIA), a measurement method by simple immunochromatography, high performance liquid chromatography (HPLC), an electrophoresis method, a capillary electrophoresis method, capillary chip electrophoresis method, a mass spectrometry method, a measurement method based on an immunoagglutination method, such as an immunonephelometry method or an immunoturbidimetry method, and an immunoblotting method.

It is noted that the proHpt amount may not be the actual amount of proHpt (the amount of the proHpt protein). It may be a relative value (in terms of the relative unit) of a measured value of proHpt with respect to a reference value which is a measured value (a signal value such as fluorescence intensity, absorbance, or the like) measured by carrying out measurement using a purified proHpt of which the concentration is known, where the measured value of proHpt is obtained by carrying out the same measurement using a subject-derived specimen.

Specific examples of the measurement method for the proHpt amount include the following method A, method B, and method C.

Method A: Method including the following steps (A-1) to (A-4):
(A-1) the step of subjecting a subject-derived specimen to a reduction treatment,
(A-2) the step of separating proHpt from the specimen obtained in the step (A-1),
(A-3) the step of bringing the proHpt obtained in the step (A-2) into contact with the antibody 1 to form a complex of the proHpt and the antibody 1, and
(A-4) the step of measuring an amount of the complex obtained in the step (A-3) to measure the proHpt amount.

Method B: Method including the following steps (B-1) to (B-4):
(B-1) the step of subjecting a subject-derived specimen to a reduction treatment,
(B-2) the step of bringing the specimen obtained in the step (B-1) into contact with the antibody 1 to form a complex of the proHpt in the specimen and the antibody 1,
(B-3) the step of separating the complex obtained in the step (B-2), and
(B-4) the step of measuring an amount of the complex separated in the step (B-3) to measure the proHpt amount.

Method C: Method including the following steps (C-1) to (C-3):
(C-1) the step of subjecting a subject-derived specimen to a reduction treatment,
(C-2) the step of bringing the specimen obtained in the step (C-1) into contact with the antibody 1 and an antibody 2, which is an antibody that recognizes a β chain of proHpt, to form a complex of the proHpt in the specimen, the antibody 1, and the antibody 2, and
(C-3) the step of measuring an amount of the complex obtained in the step (C-2) to measure the proHpt amount.

Each of the methods will be described below.

### [Method A]

As a specific method of the method A, for example, there is a method according to a Western blotting method.

For example, a subject-derived specimen is subjected to a reduction treatment, according to the above-described method, to dissociate in Hpt in the specimen into the α chain and the β chain.

Next, the specimen subjected to the reduction treatment is subjected to electrophoresis such as SDS-PAGE, isoelectric point electrophoresis, or two-dimensional electrophoresis according to a conventional method to separate proHpt from other proteins in the specimen, and then the proHpt in the gel is transferred (blotted) and immobilized on a blot membrane such as a PVDF (polyvinylidene difluoride) membrane or a nitrocellulose membrane according to a conventional method of Western blotting. Examples of the transfer method include a method such as electric transfer.

The blot membrane after the transfer is impregnated with a solution containing the antibody 1 (a primary antibody) according to the embodiment of the present invention, and the proHpt on the blot membrane is reacted with the antibody 1 to form a complex of the proHpt and the antibody 1. Next, the blot membrane is impregnated with a solution containing a labeled antibody (a labeled secondary antibody) labeled with a measurable labeling substance to cause a reaction to occur, whereby a complex of the proHpt, the antibody 1, and the labeled secondary antibody is formed on the blot membrane. Next, a proper color developing substrate depending on the labeling substance of the labeled antibody is reacted to detect a migration fraction. From the migration position of the migration fraction, a fraction having a molecular weight of 57 kDa is defined as a separation fraction of proHpt, and an amount of the fraction derived from the labeling substance or a protein amount is measured and used as the proHpt amount in the subject-derived specimen.

### [Method B]

As a specific method of the method B, for example, there is a method according to a Western blotting method or an ELISA method.

The method B will be described as below, taking a method of carrying out the method B according to a Western blotting method, as an example.

For example, a subject-derived specimen is subjected to a reduction treatment, according to the above-described method, to dissociate in Hpt in the specimen into the α chain and the β chain. The specimen subjected to the reduction treatment is reacted with the antibody 1 (a primary antibody) according to the embodiment of the present invention to form a complex of the proHpt in the specimen and the antibody 1.

Next, the obtained specimen is subjected to electrophoresis such as SDS-PAGE, isoelectric point electrophoresis, or two-dimensional electrophoresis according to a conventional method to separate the complex from other proteins in the specimen. Next, the complex in the gel is transferred (blotted) and immobilized on a blot membrane such as a PVDF (polyvinylidene difluoride) membrane or a nitrocellulose membrane according to a conventional method of Western blotting.

The blot membrane after the transfer is impregnated with a solution containing a labeled antibody (a labeled secondary antibody) labeled with a measurable labeling substance to cause a reaction to occur, whereby a complex of the proHpt, the antibody 1, and the labeled secondary antibody is formed on the blot membrane. Next, a proper color developing substrate that matches with the labeling substance of the labeled antibody is reacted to detect a migration fraction. From the migration position of the migration fraction, a fraction having a molecular weight of 57 kDa is defined as a separation fraction of proHpt, and an amount of the fraction derived from the labeling substance or a protein amount is measured and used as the proHpt amount in the subject-derived specimen.

The method of the reduction treatment carried out in the method A and the method B is as described in the section of "(I) Reduction treatment" described above, and the same apples to specific examples and preferred examples of the reagents that are used therein, and specific examples and preferred examples of the treatment conditions and the like.

The antibody 1 (a primary antibody) according to the embodiment of the present invention which is used in the method A and the method B is as described in the section of " (II) Antibody 1 according to embodiment of present invention", the same applies to preferred examples, specific examples, and the like thereof. In addition, the antibody that is used as the labeled secondary antibody is an antibody that recognizes an antibody derived from an animal species from which the antibody 1 (a primary antibody) has been prepared. Examples of the labeling substance of the labeled secondary antibody include the same one as the labeling substance for labeling the antibody 1 according to the embodiment of the present invention or the antibody 2 according to the embodiment of the present invention, which will be described in the method C described later, and the same applies to preferred examples, specific examples, and the like thereof.

In addition, the same applies to specific examples and preferred examples of the labeling method for the labeling substance and the measurement method for the label. In order to detect the labeling substance, a commercially available luminescence reagent for Western blotting (ImmunoStar Zeta, manufactured by FUJIFILM Wako Pure Chemical Corporation) or the like may be used.

The method of measuring proHpt according to the method A or the method B will be described as below, taking the method A as an example.

A subject-derived specimen is mixed with a buffer solution containing 20 w/v% 2-mercaptoethanol and purified water to be subjected to a reduction treatment. Next, the obtained specimen is subjected to SDS-PAGE according to a conventional method. The obtained migration gel is blotted and immobilized on a blot membrane such as a PVDF (polyvinylidene difluoride) membrane or a nitrocellulose membrane according to a conventional method of Western blotting.

The blot membrane after the transfer is impregnated with a buffer solution containing the antibody 1 (for example, 10-7G2A) according to the embodiment of the present invention. Next, it is impregnated with a solution containing a peroxidase-labeled anti-mouse IgG antibody. Next, the blot membrane after the reaction is washed with a buffer solution such as PBS-T and then immersed in a solution containing a peroxidase luminescence substrate such as ImmunoStar Zeta (manufactured by FUJIFILM Wako Pure Chemical Corporation) to develop a color. The blot membrane after the color development is washed with purified water or the like to terminate the reaction. The luminescence signal value of each fraction is detected, and then a fraction having a molecular weight of 57 kDa corresponding to proHpt is determined as a proHpt fraction. The amount of the fraction derived from the labeling substance is measured. Separately, the same measurement is carried out using a specimen containing proHpt having a known concentration. A ratio of an amount derived from a labeling substance, obtained by a measurement using a subject-derived specimen, to an amount derived from a labeling substance, obtained by carrying out the same measurement using a specimen containing proHpt having a known concentration, is defined as the amount of the proHpt in the subject-derived specimen.

### [Method C]

Specific examples of the method C include an immunological measurement method using the antibody 1 according to the embodiment of the present invention and an antibody (the antibody 2) that binds to the β chain of proHpt.

That is, a subject-derived specimen is subjected to a reduction treatment, according to the above-described method, to dissociate in Hpt in the specimen into the α chain and the β chain.

The specimen subjected to the reduction treatment is brought into contact with the antibody 1 according to the embodiment of the present invention and the antibody that binds to the β chain of proHpt to form a complex of the antibody 1, the proHpt, and the antibody that binds to the β chain of proHpt. The amount of the obtained complex is measured. With the above method, proHpt can be specifically measured.

The method of the reduction treatment carried out in the method C is as described in the section of "(I) Reduction treatment" described above, and the same apples to specific examples and preferred examples of the reagents that are used therein, and specific examples and preferred examples of the treatment conditions and the like.

As the antibody that recognizes the β chain of proHpt, which is used in Method C, it is also possible to use an antibody that binds to the β chain of Hpt. An antibody that specifically recognizes only the β chain of proHpt or an antibody that specifically recognizes only the β chain of Hpt is preferable.

Hereinafter, the antibody will be referred to as "the antibody 2 according to the embodiment of the present invention" or simply "the antibody 2".

In addition, the antibody 2 according to the embodiment of the present invention may be a monoclonal antibody or a polyclonal antibody. A monoclonal antibody is more preferable. It may be a commercially available product or an antibody appropriately prepared according to a conventional method. In addition, it is optional that these are used alone or in a combination thereof.

In addition, the antibody 2 may be an antigen-binding fragment of the antibody 2, and specific examples thereof include fragments of the antibody 2, such as Fab, Fab', F(ab')₂, Fv, Fd, single chain Fv (scFv), disulfide bonded Fv (sdFv), VL, VH, a diabody ((VL-VH)₂ or (VH-VL)₂), a triabody (a trivalent antibody), a tetrabody (a tetravalent antibody), a minibody ((scFV-CH₃)₂), IgG-delta-CH₂, scFv-Fc, and (scFv)₂-Fc, which specifically recognize the β chain of proHpt.

The origin of the antibody 2 is not particularly limited; however, the anti-PSMA antibody is an antibody that is derived from, for example, a rabbit, a rat, a mouse, a sheep, a goat, or a horse, and has the properties described above. A commercially available product or antibody obtained, for example, according to a method described in "Introduction to Immunological Experiments, 2nd Printing, Nao Matsuhashi et al., Japan Scientific Societies Press, 1981" or the like, may be used, each of which has the properties described above.

According to a method of producing a polyclonal antibody or a method of producing a monoclonal antibody according to a conventional method, the antibody 2 can be obtained by immunizing an animal with proHpt, the β chain of Hpt, or a fragment thereof and then collecting, purifying, and screening an antibody produced in vivo.

The Hpt that is used as an antigen can be obtained by being extracted from a culture solution or culture supernatant of a cancer cell line according to a conventional method, for example, according to a method using an anti-Hpt antibody column. A commercially available product may be used.

In addition, the proHpt (full length) that is used as an antigen can be obtained, according to a previous report (Mi-Kyung Oh, et al., FEBS letters vol. 589, pp. 1009-1017, 2015), by constructing an expression vector of Hpt in which an amino acid mutation has been introduced in a portion to be cleaved by C1RL, overexpressing the Hpt in a proper host cell such as HEK293 cells, subsequently recovering the culture supernatant, and then carrying out recovery and purification by affinity column chromatography using an Hpt-binding carrier.

The proHpt, the β chain of Hpt, or a fragment thereof, which is used as an antigen, can be produced, for example, by a conventional chemical synthesis method according to the amino acid sequence thereof.

A commercially available recombinant product of the β chain subunit may be used. Examples thereof include Recombinant Human Haptoglobulin beta protein (ab63120) (manufactured by Abcam plc.).

A commercially available antibody 2 may be used. Examples thereof include Haptoglobin (5C5) Monoclonal Antibody (manufactured by Bioss Inc., a rabbit anti-human haptoglobin antibody, IgG, Catalog No. bsm-52899R).

In the method C, examples of the method of measuring the complex of the antibody 1 according to the embodiment of the present invention, the proHpt, and the antibody 2 according to the embodiment of the present invention include the above-described methods such as the enzyme immunoassay (EIA), the capillary electrophoresis method, and the capillary chip electrophoresis method.

Examples of the principle of these measurements include a sandwich method, a competitive method, and a two-antibody method, and, and a sandwich method is preferable. Further, it is also possible to carry out measurement by a heterogeneous method in which the B/F separation is carried out using an insoluble carrier or the like, or it is also possible to carry out measurement by a homogeneous method in which the B/F separation is not carried out.

### - Sandwich method -

In the method C, examples of the measurement method according to the sandwich method include a method in which a subject-derived specimen, the antibody 1, and the antibody 2 are brought into contact with each other to form a complex of the proHpt, the antibody 1, and the antibody 2, and the amount of the complex is measured.

It is preferable that the antibody 1 and/or the antibody 2, which are used in the measurement method according to the sandwich method, is labeled with a labeling substance or the like. For example, in a case where the antibody 1 (the labeled antibody 1) labeled with a labeling substance is used as the antibody 1 according to the embodiment of the present invention, a complex 1 may be measured based on the amount of the labeling substance of the labeled antibody 1, and for example, in a case where the antibody 2 (the labeled antibody 2) labeled with a labeling substance is used as the antibody 2, the complex 1 or a complex 2 may be measured based on the amount of the labeling substance of the labeled antibody 2.

Examples of the labeling substance that is used for labeling the antibody 1 or the antibody 2 include enzymes that are used in the general immunoassay, such as peroxidase (POD), microperoxidase, alkaline phosphatase, β-galactosidase, glucose oxidase, glucose-6-phosphate dehydrogenase, acetylcholinesterase, malate dehydrogenase, and luciferase; radioactive isotopes that are used in the radioimmunoassay (RIA), such as 99mTc, 131I, 125I, 14C, 3H, 32P, and 35S; fluorescent substances that are used in the fluoroimmunoassay (FIA), such as fluorescein, dansyl, fluorescamine, coumarin, naphthylamine, fluorescein isothiocyanate (FITC), rhodamine, rhodamine X isothiocyanate, sulforhodamine 101, lucifer yellow, acridine, acridine isothiocyanate, riboflavin, and derivatives thereof; luminescent substances such as luciferin, isoluminol, luminol, and a bis(2,4,6-trifluorophenyl)oxalate; substances having absorption in an ultraviolet region such as phenol, naphthol, anthracene, and derivatives thereof; labeling substances such as substances having properties as spin labeling agents represented by compounds having an oxyl group such as 4-amino-2,2,6,6-tetramethylpiperidine-1-oxyl, 3-amino-2,2,5,5-tetramethylpyrrolidine-1-oxyl, and 2,6-di-t-butyl-α-(3,5-di-t-butyl-4-oxo-2,5-cyclohexadien-1-ylidene)-p-tolyloxyl; HiLyte coloring agents such as HiLyte Fluor 647, HiLyte Fluor 488, HiLyte Fluor 555, HiLyte Fluor 680, and HiLyte Fluor 750 (all of which are trade names of HiLyte Bioscience, Inc.); Alexa coloring agents such as Alexa Fluor Dye 350, Alexa Fluor Dye 430, Alexa Fluor Dye 488, Alexa Fluor Dye 532, Alexa Fluor Dye 546, Alexa Fluor Dye 555, Alexa Fluor Dye 568, Alexa Fluor Dye 594, Alexa Fluor Dye 633, Alexa Fluor Dye 647, Alexa Fluor Dye 660, Alexa Fluor Dye 680, Alexa Fluor Dye 700, and Alexa Fluor Dye 750 (all of which are trade names of Molecular Probes, Inc.); CyDye coloring agents such as Cy3, Cy3.5, Cy5, Cy5.5, and Cy7 (all of which are trade names of Amersham Biosciences, Inc.); and coloring agents such as Coomassie Brilliant Blue R250 and Methyl Orange. Among them, enzymes such as peroxidase, microperoxidase, alkaline phosphatase, β-galactosidase, glucose oxidase, glucose-6-phosphate dehydrogenase, acetylcholinesterase, malate dehydrogenase, and luciferase are preferable, and peroxidase is more preferable.

In addition, in order to bind (label) the same labeling substance as described above to the antibody 1 or the antibody 2, it is sufficient to appropriately use, for example, a labeling method known per se generally used in the immunoassay such as EIA, RIA, or FIA, which is known per se.

In addition, since various kits for binding (labeling) such a labeling substance as described above to a protein are also commercially available, the labeling of the antibody 1 or the antibody 2 may be carried out using the kit according to the instruction manual attached to the kit.

In addition, in a case of carrying out high performance liquid chromatography, a capillary electrophoresis method, or a capillary chip electrophoresis method, a separation improving substance such as a nucleic acid such as DNA or RNA may be bound in order to more clearly separate the antigen-antibody complex from the liberated labeled antibody (JP3070418B, JP3531372B, or the like).

It is noted that the solution containing the labeled antibody 1 or the labeled antibody 2, used for the measurement, may contain those that are generally used as stabilizers in this field, for example, sugars, a protein, and a surfactant, within a concentration range that is generally used in this field.

In a case of labeling the antibody 1 and/or the antibody 2 as described above, it is necessary to separate the antibody (the labeled antibody) labeled with the liberated labeling substance and the complex. Therefore, for example, in a case of forming the complex 1, it is preferable to label any antibody of the antibody 1 or the antibody 2 with a labeling substance and to immobilize the remaining unlabeled antibody 1 or the antibody 2 on an insoluble carrier. In this case, it is preferable to immobilize the antibody 1 on an insoluble carrier and to label the antibody 2 with a labeling substance. Regarding the separation of the liberated labeled antibody and the complex, they can be separated according to a known B/F separation method.

As the insoluble carrier for immobilizing the antibody 1 or the antibody 2, any insoluble carrier can be used as long as it is an insoluble carrier that is used in a general immunological measuring method and the like. Specific examples thereof include synthetic polymer compounds such as polystyrene, polypropylene, polyacrylic acid, polymethacrylic acid, polyacrylamide, polyglycidyl methacrylate, polyvinyl chloride, polyethylene, polychlorocarbonate, a silicone resin, and silicone rubber, and inorganic substances such as porous glass, frosted glass, ceramics, alumina, silica gel, activated carbon, and a metal oxide. In addition, these insoluble carriers can be used in various and diverse forms such as a microtiter plate, a bead, a tube, a dedicated tray in which a large number of tubes are integrally molded, a disc-shape piece, and a fine particle (a latex particle). Among them, a microplate or a bead is preferable from the viewpoints of ease of washing, operability in simultaneous processing of a large number of specimens (samples), and the like.

The method of immobilizing the antibody 1 or the antibody 2 according to the embodiment of the present invention on an insoluble carrier is not particularly limited as long as the method is carried out according to a method generally used in this field. Examples thereof include all of the immobilization methods known per se, which are generally used in this field, examples of which include a chemical bonding method (a method of carrying out immobilization with a covalent bond) and a method of carrying out physical adsorption.

Preferred examples thereof include a method in which a solution containing the antibody 1 or the antibody 2 in a range of generally 0.1 µg/mL to 20 mg/mL and preferably 1 µg/mL to 5 mg/mL is brought into contact with an insoluble carrier at a proper temperature and reacted for a predetermined time to obtain an insoluble carrier (a solid phase) to which the antibody 1 or the antibody 2 is bound.

The solvent for preparing the solution of the antibody 1 or the antibody 2 may be any one as long as it does not have a property of hindering the antibody 1 or the antibody 2 from adsorbing or binding to an insoluble carrier. For example, purified water, a buffer solution having a buffering action, for example, at a pH of 5.0 to 10.0 and preferably in the vicinity of the neutral pH of 6.5 to 8.5 (for example, a phosphate buffer solution, a Tris buffer solution, a Good buffer solution, a glycine buffer solution, a borate buffer solution, or the like) is preferable. In addition, the concentration of the buffering agent in these buffer solutions is appropriately selected generally from a range of generally 10 mM to 500 mM and preferably 10 mM to 300 mM. In addition, this solution may contain, for example, sugars, salts such as NaCl, a surfactant, a preservative, and a protein, as long as the amount thereof does not hinder the antibody 1 or the antibody 2 from adsorbing or binding to an insoluble carrier.

In addition, from the viewpoint of preventing a non-specific reaction during measurement, it is desirable to carry out a blocking treatment that is generally carried out in this field, that is, a treatment in which an insoluble carrier to which the antibody 1 or the antibody 2 obtained as described above is bound is further immersed in a solution containing a protein unrelated to proHpt or Hpt, for example, human serum albumin, bovine serum albumin, a milk protein such as skim milk, egg albumin, or a commercially available blocking agent (for example, Block Ace (manufactured by DS Pharma Biomedical Co., Ltd.)).

In addition, it is also possible to immobilize the antibody 1 or the antibody 2 on an insoluble carrier by using a very firm binding reaction such as the avidin-biotin reaction that is used in this field.

Furthermore, as described above, the insoluble carrier on which the antibody 1 or the antibody 2 is immobilized can also be used in an immunonephelometry method and an immunoagglutination method, which are known per se.

The method of measuring the label amount in the complex generated as a result of the reaction using the labeled antibody 1 or the labeled antibody 2 differs depending on the kind of labeling substance. However, it may be carried out according to each predetermined method depending on the properties possessed by the labeling substance, which can be detected by some kind of methods. For example, in a case where the labeling substance is an enzyme, the measurement may be carried out according to the enzyme immunoassay. In a case where the labeling substance is a radioactive substance, the measurement may be carried out, for example, according to a common method that is carried out in RIA, by appropriately selecting and using a measurement instrument such as an immersion type GM counter, a liquid scintillation counter, a well type scintillation counter, or a counter for HPLC, depending on the kind and the intensity of radiation generated by the radioactive substance. In a case where the labeling substance is a fluorescent substance, the measurement may be carried out according to a conventional method that is carried out in FIA using a measurement instrument such as a fluorophotometer. In a case where the labeling substance is a luminescent substance, the measurement may be carried out according to a conventional method using a measurement instrument such as a photo counter. In a case where the labeling substance is a substance having absorption in the ultraviolet region, the measurement may be carried out according to a conventional method using a measurement instrument such as a spectrophotometer. In a case where the labeling substance has a spin property, the measurement may be carried out according to a conventional method using an electron spin resonance apparatus.

Further, in a case where the labeling substance is an enzyme, examples of the measurement include a method known per se, such as a method of reacting the enzyme with a color developing reagent to induce a color developing reaction and then measuring the amount of a coloring agent generated as a result of the reaction with a spectrophotometer or the like. It is noted that in order to stop the color developing reaction, a reaction terminating method that is generally carried out in the related field may be used, where the reaction terminating method includes adding to the reaction solution, for example, an enzyme activity inhibitor such as 1 to 6 N sulfuric acid or a reaction terminating agent attached to the kit.

In addition, examples the color developing reagent include color developing reagents generally used in the related art, such as tetramethylbenzidine (TMB), 3,3',5,5'-tetramethylbenzidine (TMBZ), o-phenylene diamine, o-nitrophenyl-β-D-galactoside, 2,2'-azino-bis (3-ethylbenzthiazoline-6-sulfonic acid) (ABTS), N-ethyl-N-sulfopropyl-m-anisidine (ADPS), and p-nitrophenyl phosphate. In addition, the using concentrations of these color developing reagents may be appropriately set from the concentration range generally used in this field.

In addition, in order to stop the color developing reaction, a reaction terminating method that is generally carried out in the related field may be used, where the reaction terminating method includes adding to the reaction solution, for example, an enzyme activity inhibitor such as 1 to 6 N hydrochloric acid.

In addition, examples of the method of measuring proHpt using only an unlabeled antibody include a method of carrying out measurement using properties derived from the obtained complex, specifically, a method of measuring an enzyme activity such as a protease activity or a fluorescence deflection degree as an absorbance, which is possessed by the complex itself, and a method of a homogeneous immunoassay system such as surface plasmon resonance.

The concentrations of the antibody 1 and the antibody 2 which are used in the measurement method for the proHpt amount according to the embodiment of the present invention may be appropriately set in a range generally used in this field, depending on the measurement method.

All the reagent to be used in the method C, the concentration thereof at the time of measurement, and the measurement conditions and the like (such as the reaction temperature, the reaction time, the pH at the time of reaction, the measurement wavelength, and measurement device) at the time of carrying out the measurement may be appropriately selected according to the measurement operation method of the same immunological measuring method as described above, which is a method known per se. Any automatic analyzer, any spectrophotometric system, and the like to be used, which are generally used in this field, can be used without exception.

The solvent that is used for the solutions of the antibody 1 and the antibody 2 which are used in the method C is preferably a buffer solution. The buffer solution is not particularly limited as long as it is generally used in this field; however, examples thereof include a buffer solution having a buffering action at a pH of generally 5.0 to 10.0 and preferably in the vicinity of the neutral pH of 6.5 to 8.5. Specific examples thereof include all buffers that are used in measurement methods generally using an antigen-antibody reaction, such as a Tris buffer solution, a phosphate buffer solution, a veronal buffer solution, a borate buffer solution, and a Good buffer solution. The concentration of the buffering agent of these buffer solutions is appropriately selected generally from a range of generally 10 mM to 1,000 mM and preferably 10 mM to 300 mM. In addition, the pH thereof is not particularly limited as long as it does not suppress the antigen-antibody reaction; however, it is generally preferably in a range of 5 to 9.

Examples of the method of measuring the proHpt amount according to the sandwich method using the antibody 1 and the antibody 2 according to the embodiment of the present invention include the following method.

In a case where a specimen subjected to a reduction treatment is reacted with the antibody 1 according to the embodiment of the present invention, the antibody 1 binds to the α chain of proHpt. Next, in a case where an antibody (the antibody 2 according to the embodiment of the present invention) that recognizes the β chain of proHpt is reacted with the labeled antibody 2 labeled with a labeling substance, the labeled antibody 2 binds to the β chain of proHpt. Then, the proHpt amount in the test specimen can be obtained by measuring the amount of the obtained complex of the antibody 1-proHpt-labeled antibody 2.

Examples of another method of measuring the proHpt amount according to the sandwich method include the following method. That is, in a case where a specimen subjected to a reduction treatment is reacted with an antibody (the antibody 2) that recognizes the β chain of proHpt, the antibody 2 binds to the β chain of proHpt. Next, in a case where the antibody 1 (the antibody 1) according to the embodiment of the present invention is reacted with the labeled antibody 1 labeled with a labeling substance, the labeled antibody 1 binds to the α chain of proHpt. Then, the proHpt amount in the subject-derived specimen can be obtained by measuring the amount of the obtained complex of the labeled antibody 1-proHpt-labeled antibody 2.

A specific example of the method C will be described as below, taking a case where a proHpt concentration in a specimen is measured by using peroxidase (POD) as a labeling substance and then using the antibody 1 according to the embodiment of the present invention, immobilized on an insoluble carrier, and the antibody 2 labeled with POD, as an example.

First, a reducing agent solution is prepared by adding a reducing agent (for example, dithiothreitol) to a proper buffer solution (for example, a Good buffer solution such as a 0.1 M Tris buffer solution) to have a proper concentration (generally about 500 mM). Then, after adding the reducing agent solution (final concentration: about 50 mM) to a subject-derived specimen (for example, serum) containing proHpt, heating is carried out at about 0°C to 120°C for about 1 minute to 100 minutes (generally about 30 minutes) to carry out a reduction treatment.

Next, the subject-derived specimen subjected to the reduction treatment is brought into contact with an insoluble carrier (containing 0.1 ng to 0.1 mg of the antibody 1 according to the embodiment of the present invention) on which the antibody 1 according to the embodiment of the present invention (for example, 10-7G2A) has been immobilized, and reacted at 4°C to 40°C for 3 minutes to 20 hours to generate, on the insoluble carrier, a complex of the antibody 1 according to the embodiment of the present invention and the proHpt, and a complex of the antibody 1 according to the embodiment of the present invention and the α chain of Hpt, where the α chain has been dissociated by the reduction treatment. Next, in a case where a general washing treatment is carried out, the β chain of Hpt, dissociated by the reduction treatment, is removed. Then, 10 to 100 µL of a solution (containing 0.01 ng to 0.1 mg of the antibody 2) containing the antibody 2 labeled with POD is reacted at 4°C to 40°C for 3 minutes to 20 hours to generate a complex of the antibody 1-proHpt-labeled antibody 2 on the insoluble carrier. Subsequently, a color developing solution such as a TMBZ solution is added and then reacted for a certain period of time, and a reaction termination solution such as 1N HCl is added to terminate the reaction, and then, the absorbance at a wavelength of 450 nm is measured.

On the other hand, the proHpt having a known concentration is subjected to the same operation as described above by using the same reagent as described above to create a calibration curve of the measured value and the concentration. The measured value obtained in the above measurement is applied to the calibration curve to determine the amount of proHpt.

Alternatively, the proHpt having a known concentration is subjected to the same operation by using the same specimen as described above to measure the absorbance in the same manner. The ratio of the absorbance obtained by using the subject-derived specimen to the absorbance obtained by using the proHpt having a known concentration may be determined, and this value may be defined as the relative value (in terms of the relative unit) of the proHpt concentration in the subject-derived specimen.

### - Capillary electrophoresis method -

A method of measuring the proHpt amount according to the method C using the capillary electrophoresis method will be described below.

In order to measure proHpt with the capillary electrophoresis method or the capillary chip electrophoresis method, a subject-derived specimen subjected to a reduction treatment by the method described above is used, and the electrophoresis method may be carried out in accordance with the method described, for example, in J. Chromatogr. 593 253-258 (1992), Anal. Chem. 64, 1926-1932 (1992), or WO2007/027495.

### Specific examples thereof include the following method.

That is, a subject-derived specimen is subjected to a reduction treatment, according to the above-described method, to dissociate in Hpt in the specimen into the α chain and the β chain.

Next, the specimen subjected to the reduction treatment, the antibody 1 according to the embodiment of the present invention labeled with a labeling substance, and an antibody (the antibody 2) that recognizes the β chain of proHpt are mixed with each other and reacted for 5 to 30 minutes and preferably for 10 seconds to 15 minutes at a temperature kept to 25°C to 40°C. As a result of the reaction, a complex of the proHpt and the labeled antibody 1, the complex 2 of the α chain of Hpt generated in the reduction treatment and the antibody 1, and a complex 3 of the β chain of Hpt generated in the reduction treatment and the antibody 2 are generated. Then, the resulting complex 1, complex 2, and complex 3 are separated, for example, by capillary chip electrophoresis, and the amount derived from the labeling substance is measured, for example, with a fluorescence detector or the like. The obtained measured value derived from the complex 1 is applied to the calibration curve which indicates the relationship between the measured value and the proHpt concentration and which has been created in advance by using the proHpt solution having a known concentration, whereby the proHpt concentration in the specimen can be determined.

Examples of another method of measuring proHpt according to an immunological measurement method include the following method D.

Method D: Method including the following steps (D-1) to (D-4):
(D-1) the step of subjecting a subject-derived specimen to a reduction treatment,
(D-2) the step of bringing the specimen obtained in the step (D-1), the antibody 1, an antibody 1-2 which is an antibody that recognizes an α chain of proHpt, the antibody 1-2 having an epitope different from an epitope recognized by the antibody 1, and an antibody 2 which is an antibody that recognizes a β chain of proHpt, into contact with each other, to form a complex of proHpt, the antibody 1, the antibody 1-2, and the antibody 2,
(D-3) the step of separating the complex of the proHpt, the antibody 1, the antibody 1-2, and the antibody 2, where the complex is obtained in the step (D-2), and
(D-4) the step of measuring an amount of the complex separated in the step (D-3) to measure the proHpt amount.

The method D is a method of measuring proHpt by further using, in combination, an antibody (the antibody 1-2) that recognizes the α chain of proHpt, where the antibody has an epitope different from an epitope recognized by the antibody 1 according to the embodiment of the present invention, in the method C.

Specific examples of the method D include capillary electrophoresis and capillary chip electrophoresis.

That is, a subject-derived specimen is subjected to a reduction treatment, according to the above-described method, to dissociate in Hpt in the specimen into the α chain and the β chain.

Next, the specimen subjected to the reduction treatment, the antibody 1 labeled with a labeling substance, an antibody (the antibody 2) that recognizes the β chain of proHpt, and an antibody (the antibody 1-2) that recognizes the α chain of proHpt are mixed with each other and reacted for 5 to 30 minutes and preferably for 10 seconds to 15 minutes at a temperature kept to 25°C to 40°C. As a result of the reaction, a complex of the proHpt, the labeled antibody 1, the antibody 2, and the antibody 1-2, the complex 2 of the α chain (α1 and α2) of Hpt generated in the reduction treatment, the labeled antibody 1, and the antibody 1-2, and the complex 3 of the β chain of Hpt generated in the reduction treatment and the labeled antibody 2 are generated. Then, the resulting complex 1, complex 2, and complex 3 in the obtained reaction solution are separated, for example, by capillary chip electrophoresis, and the amount derived from the labeling substance is measured, for example, with a fluorescence detector or the like. The obtained measured value derived from the complex 1 is applied to the calibration curve which indicates the relationship between the measured value and the proHpt concentration and which has been created in advance by using the proHpt solution having a known concentration, whereby the proHpt concentration in the subject-derived specimen can be determined.

The method of the reduction treatment carried out in the method D is as described in the section of "(I) Reduction treatment" described above, and the same apples to specific examples and preferred examples of the reagents that is used therein, and specific examples and preferred examples of the treatment conditions and the like.

It suffices that the antibody 1-2 that is used in the measurement of the proHpt according to the embodiment of the present invention is an antibody that recognizes the α chain of proHpt, where the antibody 1-2 has an epitope different from an epitope recognized by the antibody 1 according to the embodiment of the present invention, and an antibody that recognizes the α chain of Hpt can also be used. The antibody 1-2 may be a monoclonal antibody or a polyclonal antibody. A monoclonal antibody is more preferable. It may be a commercially available product or an antibody appropriately prepared according to a conventional method. In addition, it is optional that these are used alone or in a combination thereof. Hereinafter, the antibody will be referred to as "the antibody 1-2 according to the embodiment of the present invention" or simply "the antibody 1-2".

In addition, the antibody 1-2 may be an antigen-binding fragment of the antibody 1-2, and specific examples thereof include fragments of the antibody 1-2, such as Fab, Fab', F(ab')2, Fv, Fd, single chain Fv (scFv), disulfide bonded Fv (sdFv), VL, VH, a diabody ((VL-VH)2 or (VH-VL)2), a triabody (a trivalent antibody), a tetrabody (a tetravalent antibody), a minibody ((scFV-CH3)2), IgG-delta-CH2, scFv-Fc, and (scFv)2-Fc, which recognize the α chain of proHpt.

The antibody 1-2 according to the embodiment of the present invention is preferably labeled with a charged carrier molecule such as an anionic substance. Specifically, it is preferably labeled with, for example, a nucleic acid chain. In this case, the antibody 1-2 may be labeled with both the charged carrier molecule and the labeling substance according to the detection described above.

Examples of the antibody 1-2 according to the embodiment of the present invention include, as a commercially available product, Haptoglobin Mouse anti-Human Monoclonal (26E11) Antibody (manufactured by LifeSpan BioSciences. Inc, a mouse anti-human haptoglobin antibody, IgG, Catalog No. LS-C62011).

The specific examples and the like of the labeling substance and the like that labels each of the antibodies of the antibody 1, the antibody 2, and the antibody 1-2 according to the embodiment of the present invention, which are used in the capillary electrophoresis method and the capillary chip electrophoresis method, are as described in the description related to "-Sandwich method -" of "Method C" described above.

In addition, specific methods of carrying out capillary electrophoresis and capillary chip electrophoresis, and the details of reagents other than the antibody 1-2 according to the embodiment of the present invention, which are used in capillary electrophoresis and capillary chip electrophoresis, are as described in [Method C] described above.

The measurement method for proHpt according to the embodiment of the present invention is not limited to the manual method, and it may be carried out by a measurement system using an automatic analysis apparatus. It is noted that the are no particular restrictions on the combination of reagents or the like in a case of carrying out the measurement using a manual method or an automatic analysis apparatus, and a combination of reagents or the like which is considered to be the best combination may be selected and used appropriately, depending on the environment and the model of the automatic analysis apparatus to be applied, or in consideration of other factors.

### 3. Method of assisting diagnosis of inflammatory bowel disease

The method of assisting diagnosis of inflammatory bowel disease according to the present invention is a method of assisting diagnosis of inflammatory bowel disease, in which the proHpt amount in a subject-derived specimen is measured according to the measurement method described in [2. Measurement method for proHpt] described above to carry out determination based on the measurement results.

That is, using the antibody 1 according to the embodiment of the present invention, the amount of the proHpt in the subject-derived specimen is measured according to the method described in the section of [2. Measurement method for proHpt] described above, and based on the results, the proHpt-related data for determining inflammatory bowel disease (for example, information such as the presence or absence of proHpt, the concentration of proHpt, the degree of increase in the amount of proHpt, and the like) is obtained. Using the obtained data, the determination (the diagnosis and the examination) of inflammatory bowel disease is carried out, for example, by the following method.

For example, a reference value (a cutoff value) is set in advance. Separately, the data of the amount (the measured value or the like) of the proHpt in the subject-derived specimen is obtained. The obtained data is compared with the reference value to determine whether or not the amount of proHpt is equal to or larger than the reference value, and the data of the determination results is obtained. In a case where the obtained result indicates that the amount of the proHpt in the subject-derived specimen is equal to or larger than the reference value, it is possible to determine that there is a possibility or a high possibility that the subject from which the specimen has been provided suffers from inflammatory bowel disease (for example, ulcerative colitis or Crohn's disease). In a case where the measurement result (the measured value) of proHpt is lower than the reference value, it is possible to determine that there is no possibility (negative for the inflammatory bowel disease) or is a low possibility that the subject suffers from inflammatory bowel disease.

The reference value may be set based on a boundary value or the like of a value which is the proHpt amount in a specimen according to the above-described measurement method, measured by using specimens derived from a patient with inflammatory bowel disease and a patient with non-inflammatory bowel disease (non-ulcerative colitis or non-Crohn's disease). An average value of the proHpt amount of a patient with non-inflammatory bowel disease may be set as the reference value.

In addition, a plurality of determination categories may be set in accordance with the amount of the proHpt in the sample or the quantitative range thereof (the measured value or the range of the measured value) to determine inflammatory bowel disease. For example, determination categories [such as [(1) there is no risk of inflammatory bowel disease, (2) there is a low risk of inflammatory bowel disease, (3) there is a risk of inflammatory bowel disease, and (4) there is a high risk of inflammatory bowel disease] are set. Further, it is possible to determine inflammatory bowel disease by determining which determination category the measurement result of the proHpt amount of the subject-derived specimen falls into.

In addition, in the same subject, the measurement result of the proHpt in the subject-derived specimen measured at a certain time point is compared with the measurement result of the proHpt in the subject-derived specimen measured at a different time point, and the increase/decrease and/or the degree of increase/decrease in the measurement result (the measured value) is evaluated, whereby the determination can be carried out. For example, in a case where an increase in the measurement result (the measured value) is observed, it is possible to determine that there is a possibility that the pathological condition of the subject who has provided the specimen has progressed to inflammatory bowel disease. In a case where no change in the measured value of proHpt is observed, it is possible to determine that there is no change in the pathological condition of the inflammatory bowel disease of the subject. In a case where a decrease in the measurement result (the measured value) is observed, it can be determined that the pathological condition of the inflammatory bowel disease of the subject has been ameliorated.

In a case of being determined that there is a possibility or a high possibility that the patient who is the subject suffers from inflammatory bowel disease by the method of assisting determination of inflammatory bowel disease according to the present invention, it is possible to select to further carry out an invasive examination such as a colonoscopy, gastrointestinal contrast radiography, or a histopathological examination.

On the other hand, in a case of being determined that there is no possibility or is a low possibility that the patient who is the subject suffers from inflammatory bowel disease by the method of assisting determination of inflammatory bowel disease according to the present invention, it is possible to select a treatment policy of carrying out a follow-up observation as necessary without carrying out the invasive examination.

According to the method according to the embodiment of the present invention, which assists diagnosis of inflammatory bowel disease, it is possible to determine inflammatory bowel disease. In addition, it is also possible to distinguish the inflammatory bowel disease from a state of a healthy subject and colon cancer. In particular, ulcerative colitis can be distinguished from a state of a healthy subject and colon cancer.

### <4. Kit for assisting diagnosis of inflammatory bowel disease according to embodiment of present invention>

A kit for assisting diagnosis of inflammatory bowel disease according to the present invention (hereinafter, may be abbreviated as "the kit according to the embodiment of the present invention") includes, as constitutional reagents, "an antibody (the antibody 1 according to the embodiment of the present invention) that specifically binds to the amino acid sequence set forth in SEQ ID NO: 1, and a reducing agent".

The antibody 1, the antibody 1-2, and the reducing agent according to the embodiment of the present invention, which are included in the kit according to the embodiment of the present invention, are as described in the section of "<3. Method of assisting diagnosis of inflammatory bowel disease>" described above, respectively, and the same applies to the specific examples and preferred examples, and the like thereof.

In addition, the kit may further include the antibody 2 according to the embodiment of the present invention, or may further include the antibody 1-2 according to the embodiment of the present invention.

The antibody 2 and the antibody 1-2 according to the embodiment of the present invention, which are included in the kit according to the embodiment of the present invention, are as described in the section of "<3. Method of assisting diagnosis of inflammatory bowel disease>" described above, respectively, and the same applies to the specific examples and preferred examples, and the like thereof.

The antibody 1 or antibody 2 according to the embodiment of the present invention, which is included in the kit according to the embodiment of the present invention, may be bound to an insoluble carrier. The insoluble carrier, the method of binding the antibody to the insoluble carrier, the preferred examples thereof, and the like are also the same as those described in "<3. Method of assisting diagnosis of inflammatory bowel disease>" described above.

In addition, the antibody 1 or the antibody 2 according to the embodiment of the present invention may be labeled with a labeling substance. The labeling method for the labeling substance and the antibody, preferred examples thereof, and the like are also the same as those described in "<3. Method of assisting diagnosis of inflammatory bowel disease>" described above.

Further, the antibody 1-2 according to the embodiment of the present invention is labeled with a charged carrier molecule. The examples thereof are as described in the section of "<3. Method of assisting diagnosis of inflammatory bowel disease>" described above.

The concentrations of the antibody 1, the antibody 2, and the antibody 1-2 in the reagents in the kit according to the embodiment of the present invention may be appropriately set in a range generally used in this field, depending on the measurement method.

In addition, the reagent included in these kits may have those that are generally used in this field, for example, a buffering agent, a sensitizer, a surfactant, a preservative (for example, sodium azide, salicylic acid, or benzoic acid), a stabilizer (for example, albumin, globulin, water-soluble gelatin, surfactant, or sugars), an activator agent, an agent for avoiding the influence of coexisting substances, and a reagent for detecting a labeling substance, where these reagents do not inhibit the stability of coexisting reagents and the reaction or binding between the proHpt and the antibody. Further, regarding the concentration range, pH, and the like of these reagents or the like, the concentration range, pH, and the like, which are generally used in order to exhibit the effect of each of the reagents, may be appropriately selected and used.

In a case where the antibody 1 or the antibody 2 according to the embodiment of the present invention is labeled with a labeling substance, the above reagent for detecting a labeling substance is a reagent that detects the label, and it is appropriately selected depending on the kind of the labeling substance. Specific examples thereof include a substrate for measuring the absorbance such as tetramethylbenzidine or orthophenylene diamine, a fluorescent substrate such as hydroxyphenyl propionic acid or hydroxyphenyl acetic acid, a luminescent substance such as CDP-Star^{™} or luminol, a reagent for measuring absorbance such as 4-nitrophenyl phosphate, and a fluorescent substrate such as 4-methylumbelliferyl phosphate.

The preferred aspect, the specific example, the concentration, and the like of each constitutional element of the kit according to the embodiment of the present invention are as described in the section of <3. Method of assisting diagnosis of inflammatory bowel disease> described above.

Further, the kit according to the embodiment of the present invention may include, in addition to the antibody 1 and the antibody 2 according to the embodiment of the present invention, a required amount of a reagent necessary for the measurement such as the immunoassay of the proHpt amount using the antibody.

In addition, the kit according to the embodiment of the present invention may further include a reagent or the like for carrying out electrophoresis, as a constitutional element. Each reagent is as described above, and the same applies to specific examples and preferred examples thereof.

The antibody and the reagents, which constitute the kit according to the embodiment of the present invention, may each be in a solution state, a frozen state, a dried state, or a freeze-dried state.

The kit according to the embodiment of the present invention may be combined with a standard product of proHpt for creating a calibration curve that is used for measuring the proHpt. As the standard product, a commercially available standard product may be used, or a product manufactured according to a known method may be used.

Furthermore, the kit according to the embodiment of the present invention may include an instruction manual or the like for carrying out the assisting method according to the embodiment of the present invention. The "instruction manual" means a user's manual, attached document, pamphlet (leaflet), or the like for the reagents included in the kit according to the embodiment of the present invention in which the feature, the principle, the operating procedure, the determination procedure, and the like of the assisting method according to the embodiment of the present invention are substantially described in sentences and/or illustrated. Specifically, examples thereof include (i) an instruction manual in which the principle of the measurement step according to the embodiment of the present invention and the operating procedure thereof are described, and (ii) an instruction manual in which the principle, the operating procedure, and the like of the measurement step and the determination step according to the embodiment of the present invention are described.

In a case where the kit according to the embodiment of the present invention is used, the assisting method according to the embodiment of the present invention can be carried out easily, in a short time, and with high accuracy.

### <5. Device for assisting diagnosis of inflammatory bowel disease according to embodiment of present invention>

The device for assisting diagnosis of inflammatory bowel disease according to the present invention (hereinafter, abbreviated as "the assisting device according to the embodiment of the present invention") includes at least (1) a measurement unit. It may further include (2) a determination unit, (3) an output unit, and (4) an input unit.

The measurement unit in the assisting device according to the embodiment of the present invention is configured to measure the amount of the proHpt according to the embodiment of the present invention in the subject-derived specimen, where the proHpt is a biomarker. Specific examples of the assisting device include a measurement device such as a device that is used in a method according to the immunological measuring method.

It is noted that, as necessary, the measurement unit may be configured to calculate the amount of the biomarker according to the embodiment of the present invention based on the measured value.

The determination unit in the assisting device according to the embodiment of the present invention is configured to determine whether or not the measured value obtained by the measurement in the measurement unit is equal to or larger than the reference value.

Alternatively, the determination unit in the assisting device according to the embodiment of the present invention is configured to determine whether a subject suffers from inflammatory bowel disease using, as indicators, the measurement unit that measures the amount of proHpt in the subject-derived specimen obtained by the measurement unit, and the amount of the proHpt.

For example, it may be configured such that the measured value measured by the measurement unit is compared with the preset reference value (cutoff value) to determine whether it is equal to or larger than the reference value or smaller than the reference value.

The output unit in the assisting device according to the embodiment of the present invention is configured to output the result obtained by the measurement unit or/and the result obtained by the determination unit.

The input unit in the assisting device according to the embodiment of the present invention is configured to send a signal for operating the measurement unit to the measurement unit in response to an operation by an operator.

The device of the above-described (1) to (4), which constitute the assisting device according to the embodiment of the present invention, may be arranged in the same device or may be each a separate body.

In the assisting device according to the embodiment of the present invention, the subject, the subject-derived specimen, and the proHpt, which are related to the assisting device, are as described above, respectively, and the same applies to specific examples and preferred examples thereof.

The measurement, the determination, and the like carried out by the measurement unit and the determination unit of the assisting device according to the embodiment of the present invention are as described in <3. Method of assisting diagnosis of inflammatory bowel disease> described above, and the same applies to preferred examples, specific examples, and the like thereof.

In a case where the assisting device according to the embodiment of the present invention is used, the assisting method according to the embodiment of the present invention can be carried out easily, in a short time, and with high accuracy.

### <6. Method of treating inflammatory bowel disease according to embodiment of present invention>

A method of treating inflammatory bowel disease according to the present invention (hereinafter, abbreviated as "the treatment method according to the embodiment of the present invention") is carried out by measuring the amount of the biomarker according to the embodiment of the present invention in a subject-derived specimen, determining whether or not the subject suffers from inflammatory bowel disease based on the measurement result, and subjecting a patient who has been determined to be at a risk of suffering from inflammatory bowel disease or at a high risk of suffering inflammatory bowel disease to a suitable treatment based on the determination result.

The subject-derived specimen, the marker, the measurement, the determination, and the like in the treatment method according to the embodiment of the present invention are as described in the section of <3. Method of assisting diagnosis of inflammatory bowel disease> described above described above, and the same applies to preferred examples, specific examples, and the like thereof.

Specific examples of the suitable treatment in the treatment method according to the embodiment of the present invention include a surgical treatment and a therapy with a drug (mesalazine, salazosulfapyridine, infliximab, or the like).

Hereinafter, the present invention will be described in more detail with reference to Examples; however, the present invention is not limited to these Examples and the like. Examples

### Example 1. Measurement of proHpt and determination of inflammatory bowel disease

### (1) Preparation of purified proHpt

### 1) Construction of proHpt plasmid

A pcDNA3.1-Hyg (+) vector (Invitrogen, Carlsbad, USA) containing human haptoglobin genotype 2 (Hpt2) was prepared by the following method, according to the known method (Narisada M, Kawamoto S, Kuwamoto K, Moriwaki K, Nakagawa T, Matsumoto H, et al., Biochem. Biophys. Res. Commun. 2008; 377: 792-6).

In order to construct a human haptoglobin expression vector, a DNA sequence encoding an amino acid sequence in which glutamine of the amino acid 161 of the base sequence of the human haptoglobin genotype 2 (Hpt2) was substituted with arginine was designed and synthesized. It is noted that the glutamine of the amino acid 161 is a portion cleaved by C1RL.

Using the obtained DNA sequence as a template, PCR was carried out under the following conditions.
F primer: CCAAGAATCCGGCAAACCCAGTGCAGCAGATCCTGGGTGGAC (SEQ ID NO: 4)
R primer: GGCATCCAGGTGTCCACCCAGGATCTGCTGCACTGGGTTTGCC (SEQ ID NO: 5)

### PCR reaction conditions

Initial heat denaturation was carried out at 98°C for10 seconds, followed by one cycle of 10 seconds at 98°C → 2 minutes at 50°C → 8 minutes at 68°C, and then, PCR of 30 cycles was carried out under the following conditions.
Heat denaturation: 98°C, 10 seconds
Annealing: 50°C, 15 seconds
Elongation reaction: 68°C, 8 minutes

The obtained DNA fragment was subjected to ligation into the pcDNA3.1-Hyg (+) vector by using T4 DNA ligase (Promega, Madison, WI).

It was confirmed by sequencing analysis that the obtained vector contained the base sequence encoding the amino acid sequence of Hpt2 in which the above amino acid was substituted.

### 2) Purification of proHpt from HEK 293 cells

HEK293T (derived from human fetal kidney) was obtained from the American Type Culture Collection (ATCC, Manassas, VA). HEK 293T was in a moisture-containing atmosphere under the conditions of 37°C, 5% CO₂, and 95% air using a high glucose DMEM medium supplemented with 10% fetal bovine serum (heat-inactivated, HyClone, Logan, UT), 100 U/mL penicillin, and 100 µg/mL streptomycin (Nacalai Tesque, Inc.).

After culturing, the HEK293T cells were subjected to the gene introduction with the expression vector pcDNA3.1-Hyg (+) containing the amino acid-mutated Hpt2 gene, obtained in "1) Construction of proHpt plasmid" described above. After culturing for 24 hours, hygromycin B (manufactured by FUJIFILM Wako Pure Chemical Corporation) was added to the culture medium, and the transfected cells were cultured for 2 to 3 weeks to obtain a transfectant stably overexpressing proHpt.

A proHpt product was recovered from the culture supernatant of the proHpt-overexpressing HEK293T cells containing the proHpt product and purified by affinity column chromatography using a carrier to which 10-7G2A (patent application by the inventors of the present invention: PCT/JP2020/020669) was bound. The concentration of the purified proHpt was measured using a Nano Drop 1000 spectrophotometer (Thermo Fisher Scientific, Waltham, MA).

### (2) Preparation of specimen

0.25 µL of serum of each of patients with ulcerative colitis (n = 45), patients with Crohn's disease (n = 20), patients with colon cancer (n = 20), and healthy subjects (n = 20), 5 µL of a buffer solution 1 for a specimen (0.2 M Tris-HCl, pH 6.8, 8 w/v% SDS, 40 w/v% glycerol, 0.02 w/v% BPB, 20 w/v% 2-mercaptoethanol), and 14.75 µL of purified water were mixed to prepare a specimen.

A mixture obtained by mixing 1 µL of the purified proHpt (7.4 µg as proHpt) prepared in (1) described above, 15 µL of a buffer solution for a specimen, and 14.0 µL of purified water was used as a positive control.

### (3) Western blotting

20 µL of the specimen prepared in (2) described above was subjected to SDS-PAGE (7.5% gel) using a 7.5% polyacrylamide gel (manufactured by FUJIFILM Wako Pure Chemical Corporation). Using a blotting system manufactured by Bio-Rad, the obtained migration gel was blotted on a PVDF membrane in a semi-drying manner according to the protocol. The transferred PVDF membrane was subjected to a blocking treatment using 5% skim milk (0.05% TBS-T, room temperature) for 1 hour at room temperature. Next, 10-7G2A (diluted 7,500 times with 5% skim milk) was reacted at 4°C overnight. The PVDF membrane after the reaction was washed 3 times with 0.05% TBS-T and then immersed in a solution containing 5% skim milk, which was obtained by diluting a 10,000 time-diluted secondary antibody (a horseradish peroxidase-labeled anti-mouse IgG antibody (manufactured by Promega Corporation)), 200 times with a phosphate buffer solution, and reacted at room temperature for 1 hour. The PVDF membrane after the reaction was washed with PBS-T three times and then immersed in a solution containing ImmunoStar Zeta (a chemiluminescence reagent, manufactured by FUJIFILM Wako Pure Chemical Corporation) for 5 minutes to develop a color. After the color development, the PVDF membrane was washed with purified water to stop the reaction.

The luminescence signal value of each fraction was detected by using ImageQuant LAS-4000 (manufactured by GE Healthcare Japan Corporation).

The positive control was used to be subjected to the same treatment to detect a luminescence signal.

The luminescence signal value of the fraction having a molecular weight of 57,000 corresponding to proHpt was defined as the luminescence signal value of the proHpt fraction.

Further, the luminescence signal value of the fraction of the purified proHpt of the positive control fraction was defined as the luminescence signal value of the positive control.

The ratio of the luminescence signal value of the proHpt fraction obtained in the measurement using the subject-derived specimen to the luminescence signal value of the positive control fraction obtained in the measurement was determined (ProHpt (%)).

The significant test indicates the non-parametric comparison of all pairs carried out according to the Wilcoxon test, by using JMP pro 14, which is predictive analysis software.

### (4) Result

The obtained results are shown in Fig. 1.

In addition, the measurement results are shown in Table 1.

**[Table 1]**

| Disease | Number of cases of disease | Average | Standard deviation | Minimum value | Median value | Maximum value |
|---|---|---|---|---|---|---|
| Healthy subject | 20 | 1.9 | 2.0 | 0.0 | 1.5 | 7.0 |
| Ulcerative colitis | 45 | 13.2 | 12.2 | 1.2 | 11.0 | 63.1 |
| Crohn's disease | 45 | 14.8 | 10.9 | 0.0 | 13.8 | 44.8 |
| Colon cancer | 20 | 6.4 | 7.7 | 0.6 | 3.3 | 34.3 |
| Acute enteritis | 11 | 13.2 | 9.6 | 3.0 | 10.9 | 31.5 |

From Fig. 1 and Table 1, it can be seen that the proHpt amount is significantly high in a case of a patient with inflammatory bowel disease (a patient with ulcerative colitis or a patient with Crohn's disease) as compared with a case of a healthy subject and a patient with colon cancer. In addition, it can be seen from Fig. 1 that in a case of ulcerative colitis, it is significantly high with respect to a healthy subject and a patient with colon cancer.

From the above results, it is possible to distinguish inflammatory bowel disease from a state of a healthy subject and colon cancer by carrying out the determination method according to the embodiment of the present invention using 10-7G2A and using the proHpt amount as an indicator.

### Example 2. Measurement 1 of proHpt using 10-7G2A according to embodiment of present invention

### Comparative Example 1. Measurement of proHpt amount using commercially available zonulin kit

### (1) Measurement of proHpt using 10-7G2A (Example 2)

Using a serum of each of patients with ulcerative colitis (n = 59) in which the phenotype of Hpt was Hpt2-1 or Hpt2-2, the proHpt amount in the serum was measured by the same method as in Example 1.

### (2) Measurement of proHpt using commercially available zonulin measurement kit (Comparative Example 1)

By using a commercially available Human zonulin ELISA kit (manufactured by CUSABIO TECHNOLOGY LLC) including an anti-zonulin antibody, and using the serum of the patient with ulcerative colitis, which had been used in "(1) Measurement of proHpt according to present invention" described above, zonulin (another name for prohaptoglobin) was measured according to the protocol attached to the kit.

### (3) Result

Fig. 2 shows a graph in which the measured value (ng/mL) obtained in the measurement of proHpt using a commercially available zonulin measurement kit is plotted on the horizontal axis and the measured value (%) obtained in the measurement of proHpt according to the embodiment of the present invention is plotted on the vertical axis.

As revealed from Fig. 2, it is not possible to measure the proHpt amount in the serum of the patient with ulcerative colitis by using a commercially available zonulin measurement kit (all measured values were below the sensitivity); however, it is possible to measure the proHpt amount in the measurement using 10-7G2A according to the embodiment of the present invention by using the same specimen.

From the above, it can be seen that according to the measurement method for proHpt according to the embodiment of the present invention, it is possible to measure proHpt.

On the other hand, since the proHpt amount of the patient with ulcerative colitis cannot be measured even by using a commercially available zonulin measurement kit, it can be seen that the determination of ulcerative colitis cannot be carried out.

### Example 2. Measurement 2 of proHpt using 10-7G2A according to embodiment of present invention

### (1) Preparation of specimen and reagents

### 1) Specimen

0.25 µL of serum of each of patients with ulcerative colitis, patients with Crohn's disease, patients with colon cancer, and healthy subjects, 5 µL of a buffer solution 1 for a specimen (0.2 M Tris-HCl, pH 6.8, 8 w/v% SDS, 40 w/v% glycerol, 0.02 w/v% BPB, 20 w/v% 2-mercaptoethanol), and 14.75 µL of purified water were mixed to prepare a specimen.

### 2) Reagents

0.6 µg of the 10-7G2A antibody (the antibody 1 according to the embodiment of the present invention) was immobilized on one polystyrene bead, and then blocking was carried out with BSA or casein to obtain an antibody bead.

Haptoglobin (5C5) Monoclonal Antibody (manufactured by Bioss Inc., an rabbit anti-human haptoglobin antibody, IgG, Catalog No. bsm-52899R: the antibody 2 according to the embodiment of the present invention) was subjected to POD labeling and diluted with a MES buffer solution containing 2% BSA to obtain an enzyme-labeled antibody solution (0.2 nmol/L).

A solution containing 5 mmol/L luminol was used as a substrate solution, and a solution containing 0.02% hydrogen peroxide was used as a hydrogen peroxide solution.

### (2) Measurement of proHpt

The measurement was carried out by the following method using an automatic chemiluminescence enzyme immunoanalysis apparatus SphereLight Wako.

10 µL of a specimen and 130 µL of a MOPS buffer solution containing 2% BSA are added to a reaction chamber containing one antibody bead of the 10-7G2A antibody, reacted at 37°C for about 7 minutes, and washed with a phosphate buffer solution. Next, 140 µL of the enzyme-labeled antibody solution is added thereto, the reaction is carried out at 37°C for about 7 minutes, and washing is carried out with a phosphate buffer solution. Further, 70 µL of the substrate solution and 70 µL of the hydrogen peroxide solution are added thereto, and the amount of emitted light was measured.

On the other hand, the purified proHpt prepared in (1) of Example 1 is used to be diluted to 0, 0.05, 0.1, 0.5, 1, 5, 10 µg/mL with a MOPS buffer solution containing 2% BSA, and then, the measurement is carried out in the same manner as in the above method to create a calibration curve. The above measurement results were applied to the calibration curve to calculate the proHpt concentration in the specimen.

### (3) Result

Similar to Example 1, the proHpt amount was significantly high in a case of a patient with inflammatory bowel disease (a patient with ulcerative colitis or a patient with Crohn's disease) as compared with a case of a healthy subject and a patient with colon cancer. In addition, in a case of ulcerative colitis, it was significantly high with respect to a healthy subject and a patient with colon cancer.

From the above results, it has been revealed that it is possible to distinguish inflammatory bowel disease from a state of a healthy subject and colon cancer by carrying out the determination method according to the embodiment of the present invention using the antibody 1 and the antibody 2 according to the embodiment of the present invention and using the proHpt amount as an indicator.

## Claims

1. A method of assisting diagnosis of inflammatory bowel disease, the method comprising:
subjecting a subject-derived specimen to a reduction treatment and subsequently measuring a human prohaptoglobin amount in the specimen by using an antibody 1 which is an antibody that specifically binds to an amino acid sequence set forth in SEQ ID NO: 1; and
determining that a subject has inflammatory bowel disease by using the human prohaptoglobin amount as an indicator.

2. The method of assisting diagnosis of inflammatory bowel disease according to claim 1,
wherein the determination is to determine that a subject has inflammatory bowel disease in a case where the human prohaptoglobin amount is equal to or larger than a reference value.

3. The method of assisting diagnosis of inflammatory bowel disease according to claim 1,
wherein the measurement includes the following steps (A-1) to (A-4);
(A-1) the step of subjecting a subject-derived specimen to a reduction treatment,
(A-2) the step of separating human prohaptoglobin from the specimen obtained in the step (A-1),
(A-3) the step of bringing the human prohaptoglobin obtained in the step (A-2) into contact with the antibody 1 to form a complex of the human prohaptoglobin and the antibody 1, and
(A-4) the step of measuring an amount of the complex obtained in the step (A-3) to measure a human prohaptoglobin amount.

4. The method of assisting diagnosis of inflammatory bowel disease according to claim 3,
wherein the step (A-2) is carried out by an electrophoresis method.

5. The method of assisting diagnosis of inflammatory bowel disease according to claim 1,
wherein the measurement includes the following steps (B-1) to (B-4);
(B-1) the step of subjecting a subject-derived specimen to a reduction treatment,
(B-2) the step of bringing the specimen obtained in the step (B-1) into contact with the antibody 1 to form a complex of human prohaptoglobin in the specimen and the antibody 1,
(B-3) the step of separating the complex obtained in the step (B-2), and
(B-4) the step of measuring an amount of the complex separated in the step (B-3) to measure a human prohaptoglobin amount.

6. The method of assisting diagnosis of inflammatory bowel disease according to claim 5,
wherein the step (B-3) is carried out by an electrophoresis method.

7. The method of assisting diagnosis of inflammatory bowel disease according to claim 1,
wherein the measurement includes the following steps (C-1) to (C-3);
(C-1) the step of subjecting a subject-derived specimen to a reduction treatment,
(C-2) the step of bringing the specimen obtained in the step (C-1) into contact with the antibody 1 and an antibody 2, which is an antibody that recognizes a β chain of human prohaptoglobin, to form a complex of human prohaptoglobin in the specimen, the antibody 1, and the antibody 2, and
(C-3) the step of measuring an amount of the complex obtained in the step (C-2) to measure a human prohaptoglobin amount.

8. The method of assisting diagnosis of inflammatory bowel disease according to claim 7,
wherein any one of the antibody 1 or the antibody 2 is immobilized on an insoluble carrier, and the other thereof is labeled with a labeling substance.

9. The method of assisting diagnosis of inflammatory bowel disease according to claim 1,
wherein the subject-derived specimen is serum, blood plasma, or whole blood.

10. A method of obtaining data for assisting diagnosis of inflammatory bowel disease, the method comprising:
subjecting a subject-derived specimen to a reduction treatment and subsequently measuring a human prohaptoglobin amount in the specimen by using an antibody 1 which is an antibody that specifically binds to an amino acid sequence set forth in SEQ ID NO: 1.

11. An examination kit for assisting diagnosis of inflammatory bowel disease, comprising:
an antibody 1 which is an antibody that specifically binds to an amino acid sequence set forth in SEQ ID NO: 1; and
a reducing agent.

12. The examination kit for assisting diagnosis according to claim 11, further comprising an antibody 2 which is an antibody that recognizes a β chain of human prohaptoglobin.
